# EUROPÄISCHE PATENTSCHRIFT

(11) **EP 3 037 503 B1**
(45) Veröffentlichungstag und Bekanntmachung des Hinweises auf die Patenterteilung: **01.05.2024**
(21) Anmeldenummer: 14200179.1
(22) Anmeldetag: 23.12.2014
(51) Int. Cl.: C10G 3/00, C07C 1/24, C07C 29/151, C07C 41/09

(54) **VERFAHREN ZUR HERSTELLUNG UND NUTZUNG EINES KOHLENWASSERSTOFFGEMISCHES**
METHOD FOR THE CREATION AND USE OF A HYDROCARBON MIXTURE
PROCÉDÉ DE FABRICATION ET D'UTILISATION D'UN MÉLANGE D'HYDROCARBURES

(43) Veröffentlichungstag der Anmeldung: 29.06.2016
(73) Patentinhaber: GasConTec GmbH, 61348 Bad Homburg v. d. Höhe (DE)
(72) Erfinder: Wagner, Ulrich, 06406 Bernburg (DE); Wolff, Balthasar, 40833 Ratingen (DE); Müller, Dierk, 61184 Karben (DE)
(74) Vertreter: Patentanwälte Bauer Vorberg Kayser

(56) Entgegenhaltungen:
- EP-A1- 0 164 156
- WO-A2-2010/107929
- US-A- 4 481 305
- CHANG C D ET AL: "THE CONVERSION OF METHANOL AND OTHER O-COMPOUNDS TO HYDROCARBONS OVER ZEOLITE CATALYSTS", JOURNAL OF CATALYSIS, ACADEMIC PRESS, DULUTH, MN, US, Bd. 47, Nr. 2, 1. Januar 1977 (1977-01-01) , Seiten 249-259, XP009050126, ISSN: 0021-9517, DOI: 10.1016/0021-9517(77)90172-5

## Beschreibung

Die Erfindung betrifft ein Verfahren zur Herstellung und Nutzung eines Kohlenwasserstoffgemisches für Rohölraffinerien aus Erdgasen gemäß Anspruch 1. Das Kohlenwasserstoffgemisch kann ohne weitere Aufarbeitung in einer Rohöl verarbeitenden Raffinerie einer Verwendung zugeführt werden.

Es ist allgemein bekannt, dass Erdgase nach ihrer Aufspaltung in die Komponenten Wasserstoff, Kohlenmonoxid und Kohlendioxid über katalytische Syntheseschritte in Ammoniak und Methanol, aber auch petrochemische Produkte wie Benzin, Diesel oder Olefine überführt werden können. Bereits Anfang des 20. Jahrhunderts wurden die wissenschaftlichen und technischen Grundlagen für die Fischer-Tropsch-Synthese geschaffen, die Synthesegase verschiedenster Herkünfte (Kohle, Erdgas, Öl, Holz) in flüssige Kohlenwasserstoffe umwandelt. Das Verfahren wurde in vielen Schritten weiterentwickelt, großtechnisch umgesetzt und ist auch heute noch durch eine Vielzahl neuer Patente geschützt. Ein wesentlicher Nachteil der modernen Verfahren besteht darin, dass nach der Fischer-Tropsch-Synthese ein Gemisch der verschiedensten Kohlenwasserstoffe, darunter ein hoher Anteil an Wachsen, anfällt, das einer umfassenden Raffination unterzogen werden muss. Erst durch Nutzung dieser investitions- und kostenmäßig extrem intensiven Nachbehandlung können marktkonforme Produkte wie Diesel, Benzin oder Kerosin erzeugt werden.

Im Zusammenhang mit der ersten Ölkrise wurde Mitte der siebziger Jahre des vergangenen Jahrhunderts ein Alternativverfahren zur Fischer-Tropsch-Synthese entwickelt, die katalytische Konversion von Synthesegas in Methanol und nachfolgende katalytische Umwandlung (Dehydratation) des Methanols in Benzin. Dieses Verfahren, als "Methanol to Gasoline" bezeichnet, wurde ebenfalls großtechnisch umgesetzt (als MtG Technologie) und wurde/wird in verschiedenen Anlagen weltweit betrieben. Es gestattet die Produktion eines Normbenzins mit einer Oktanzahl größer 92, das die Spezifikationen der großen Märkte in den USA, Europa und Asien erfüllt. Auch dieses Verfahren erfordert nach der Dehydratation des Methanols eine Raffination/Destillation/Hydrierung, ohne die die geforderten Qualitäten des Benzins nicht erreicht werden können. Deshalb war bisher jede Anlage zur Konversion von Synthesegas in Benzin über das Zwischenprodukt Methanol gezwungen, diese Raffinationsstufe in die Gesamtanlage zu integrieren, wobei ca. 20 % der Gesamtinvestitionskosten auf diesen Prozessschritt entfallen. Ferner ist bei diesem MtG-Verfahren zum Erreichen einer vorgegebenen Oktanzahl im Benzin die Verweildauer im Reaktor zur Umwandlung des Dimethylether/Methanol/Wasser-Gemisches weit gehend fest vorgegeben und erlaubt insoweit kaum oder nur geringe Variabilität.

Das amerikanische Patent US 4,481,305 aus dem Stand der Technik beschreibt ein katalytisches Verfahren zur Vorbereitung von Kohlenwasserstoffen bei welchem ein Synthesegas mit Wasserstoff und Kohlenstoffoxiden und einem molaren Verhältnis von Kohlenstoffmonoxid zu Wasserstoff größer als 1 umgewandelt wird, wobei bei Beginn der Umwandlung ein molares Verhältnis von Kohlenstoffmonoxid zu Kohlenstoffdioxid von 5 bis 20 vorliegt.

Die europäische Patentanmeldung EP 0 164 156 aus dem Stand der Technik beschreibt ein Verfahren für die Vorbereitung einer Katalysatormischung, welches dadurch gekennzeichnet ist, dass ein Katalysator für die Entwässerung mit einer Zusammensetzung umfassend Kupfer, Zink, Chrom und/oder Aluminium vermischt wird, welche Zusammensetzung erhalten wurde ausgehend von einer wässrigen Lösung in der die Salze der betroffenen Metalle vorhanden sind.

Die internationale Patentanmeldung WO 2010/107929 A2 aus dem Stand der Technik, welche als nächstkommend angesehen wird, beschreibt, dass ein Verfahren zum katalytischen Bilden von Reaktionsprodukten das Bereitstellen einer ionischen Flüssigkeitsphase einschließen kann. Die ionische Flüssigkeitsphase kann einen Katalysator und eine ionische Flüssigkeit umfassen. Mindestens ein Reaktant kann in der ionischen Flüssigkeitsphase zur Herstellung von Reaktionsprodukten reagiert werden welche

Wesentlicher Nachteil der obigen Lösungen ist der erhebliche Aufwand zur Umwandlung des zunächst anfallenden Rohbenzins in ein marktkonformes Produkt, das in einer höchsten Anforderungen entsprechenden Methanolsynthese erzeugt werden muss.

Aufgabe der Erfindung ist es, ein vereinfachtes Verfahren zur Umwandlung von Erdgasen in Kohlenwasserstoffgemische zu schaffen, das es gestattet, die erzeugten Gemische ohne weitere Aufarbeitung in einer Rohöl verarbeitenden Raffinerie einer Verwendung zuzuführen. Einzelne Komponenten des Kohlenwasserstoffgemisches sollen dabei entsprechend der Zielrichtung der Raffination in ihren mengenmäßigen Anteilen den speziellen Anforderungen der jeweiligen Raffinerie angepasst werden können.

Die erfindungsgemäße Aufgabe wird gelöst durch ein Verfahren nach Anspruch 1. Weitere vorteilhafte Ausgestaltungen des erfindungsgemäßen Verfahrens sind in den Unteransprüchen zu Anspruch 1 angegeben.

Erfindungsgemäß ist erkannt worden, dass das Erdgas in einem vereinfachten Verfahren in ein Rohmethanol als Zwischenprodukt überführt werden kann. Dieses Rohmethanol kann dann katalytisch in ein Dimethylether-Methanol-WasserGemisch überführt werden und anschließend - ebenfalls in einem vereinfachten Verfahren - in ein Kohlenwasserstoffgemisch umgewandelt werden, welches vorzugsweise schwefel- und benzolfrei ist und nach Entwässerung und Entgasung einer Rohöl verarbeitenden Raffinerie zugeführt wird, wo es in bevorzugt spezifikationsgerechte Produkte wie Benzin bzw. Aromaten umgewandelt wird. Unter dem Begriff "Rohölraffinerie" ist also eine Rohöl verarbeitende Raffinerie zu verstehen.

Durch Variation der Reaktionsparameter, wie beispielsweise Druck, Temperatur, Verweilzeit, bei der Umwandlung des Dimethylether-Methanol-Wasser-Gemisches in ein Kohlenwasserstoffgemisch können einzelne Komponenten (Aromaten, Paraffine, Olefine) in ihren mengenmäßigen Anteilen entsprechend den speziellen Anforderungen der jeweiligen Raffinerie erhöht oder abgesenkt werden.

Das erfindungsgemäße Verfahren zur Herstellung und Nutzung eines Kohlenwasserstoffgemisches für Rohölraffinerien aus Erdgasen ist dadurch gekennzeichnet, dass das Kohlenwasserstoffgemisch wasserfrei ist. Vorzugsweise ist das Kohlenwasserstoffgemisch frei von sauerstoffhaltigen Verbindungen. Erfindungsgemäß wird das Kohlenwasserstoffgemisch aus Erdgas mit den folgenden Verfahrensschritten hergestellt: a) Umwandlung des Erdgases in ein Methanol/Wasser-Gemisch (hier und nachfolgend als "Rohmethanol" bezeichnet) ohne nachfolgende Destillation. Das erfindungsgemäße Verfahren umfasst weiter b) die katalytische Umwandlung des Rohmethanols aus Schritt a) in ein Dimethylether-Methanol-Wasser-Gemisch und anschließend c) die Umwandlung, vorzugsweise durch Dehydration, des Dimethylether-Methanol-Wasser-Gemisches aus Schritt b) in ein wasserhaltiges Kohlenwasserstoffgemisch, welches vorwiegend aus Paraffinen, C6+ Olefinen und Aromaten, insbesondere methylierten Benzolen wie z. B. Toluol und Xylol, besteht. Bevorzugt ist es, dass das wasserhaltige Kohlenwasserstoffgemisch schwefelfrei und/oder benzolfrei ist. Das erfindungsgemäße Verfahren umfasst ferner d) die Entgasung und Entwässerung des Kohlenwasserstoffgemisches, wobei das gemäß den obigen Verfahrensschritten a) bis d) erhaltene Kohlenwasserstoffgemisch nunmehr wasserfrei ist. Erfindungsgemäß wird das gemäß den obigen Verfahrensschritten a) bis d) erhaltene Kohlenwasserstoffgemisch dann einer insbesondere naheliegenden Rohöl verarbeitenden Raffinerie zugeführt, insbesondere verpumpt, und dort in insbesondere spezifikationsgerechte Produkte, vorzugsweise Benzin und/oder Aromaten, umgewandelt.

Unter dem Begriff "spezifikationsgerechte Produkte" werden hier und im Folgenden insbesondere solche petrochemischen Produkte verstanden, die - wie beispielsweise Motorenbenzin - einer Qualität entsprechen, die die Industrie oder öffentliche Aufsichtsbehörden festgelegt haben. In den Kraftstoffnormen "DIN EN 228" für Ottokraftstoffe und "DIN EN 590" sind die Mindestanforderungen an die bedeutsamsten Qualitätsmerkmale definiert, so auch an die Oktanzahlen: Normalbenzin min. 91 ROZ, Super min. 95 ROZ, Super plus min. 98 ROZ. In Deutschland dürfen Kraftstoffe nur verkauft werden, wenn sie der jeweiligen Norm entsprechen. Vorliegend wird der Begriff "Rohöl" und "Rohöl verarbeitend" synonym zu "Erdöl" bzw. "Erdöl verarbeitend" verwendet.

Unter den Begriffen "schwefelfrei" und "benzolfrei" sowie "frei von sauerstoffhaltigen Verbindungen" und "wasserfrei" wird vorzugsweise verstanden, dass das Produkt einen sehr geringen Restgehalt dieser genannten Verbindungen aufweisen kann, jedoch dahingehend nicht weiter aufgereinigt werden muss, um einer nachfolgenden Nutzung zugeführt zu werden.

Unter dem Begriff der "naheliegenden Rohöl verarbeitenden Raffinerie" wird hier und nachfolgend verstanden, dass die Rohöl verarbeitende Raffinerie in der Nähe des Ortes der Durchführung mindestens eines der Verfahrensschritte a) bis d), vorzugsweise aller dieser Verfahrensschritte, angeordnet ist. Speziell ist darunter zu verstehen, dass die Rohöl verarbeitende Raffinerie in einer Entfernung von höchstens 20 km zum Ort der Durchführung eines oder mehrerer der Verfahrensschritte a) bis d) angeordnet ist.

Bevorzugt ist es, dass das gemäß den Verfahrensschritten a) bis d) erhaltene Kohlenwasserstoffgemisch insbesondere über eine Rohrleitung zu einer Rohöl verarbeitenden Raffinerie verpumpt wird und dort je nach Konfiguration der Raffinerie entweder dem Rohöl bzw. geeigneten Spaltprodukten, z.B. aus dem "Fluidized Catalytic Cracking" (FCC), zugesetzt wird. Während der nachfolgenden Behandlung des Kohlenwasserstoffgemisches, vorzugsweise in einem Hydrotreater, wird die produzierte Menge an Benzin bzw. Benzin und Aromaten um die im gemäß den Verfahrensschritten a) bis d) erhaltene Kohlenwasserstoffgemisch enthaltene Menge Kohlenwasserstoffe erhöht.

Von großem ökonomischen und verfahrenstechnischen Vorteil ist einerseits die Vereinfachung der Technologie zur Herstellung von Rohmethanol, nämlich insbesondere der Entfall der Destillation des Rohmethanols und die damit einhergehende Reduzierung der Anforderungen an die Methanolsynthese, und andererseits der komplette Entfall der Raffination des anfallenden Kohlenwasserstoffgemisches in Verbindung mit der Methanolherstellung. So können die Investitionskosten für die Umwandlung von Erdgas in petrochemische Produkte um bis zu 35 % gesenkt werden und die technischen Voraussetzungen für die Durchführung des Verfahrens werden geringer. Da die Verarbeitungskapazität einer Rohöl verarbeitenden Raffinerie regelmäßig sehr deutlich größer ist als das Aufkommen an dem Kohlenwasserstoffgemisch aus dem erfindungsgemäßen Verfahren, kann eine solche Rohöl verarbeitende Raffinerie also erfindungsgemäß für die Verarbeitung des Kohlenwasserstoffgemisches mitbenutzt werden, ohne dass dafür eine Kapazitätserweiterung der Raffinerie erforderlich wäre. Es kann also eine bereits bestehende Infrastruktur verwendet werden, ohne dass sich dadurch deren Auslastung wesentlich verändert, und der teure Aufbau einer Raffination speziell für das Kohlenwasserstoffgemisch wird entbehrlich.

Die Destillation des Rohmethanols beim bekannten MtG-Verfahren ist aufwändig, weil bei dem Rohmethanol ein thermisches Verfahren angewandt werden muss, um das Wasser aus dem homogenen Rohmethanol trennen zu können. Bei dem erfindungsgemäßen Verfahren kann das Entfernen des Wassers unterbleiben, bis das Kohlenwasserstoffgemisch erhalten wurde. Dann kann dieses Entfernen durch eine einfache Abscheidung erfolgen, da das Wasser dann in einer getrennten Phase vorliegt. Diese Vereinfachung wiegt das dann notwendige Mitführen des Wassers durch die dazwischenliegenden Verfahrensschritte mehr als auf, zumal der Anteil des Wassers nicht übermäßig groß ist.

Ein weiterer erheblicher Vorteil ergibt sich daraus, dass entsprechend der jeweiligen Konfiguration der abnehmenden Rohöl verarbeitenden Raffinerie die Produktverteilung (Paraffine, Aromaten, Olefine) den speziellen Erfordernissen der Rohöl verarbeitenden Raffinerie angepasst werden kann. Diese Anpassung ist einfach möglich über die Einstellung der Verweilzeit im Reaktor zur Umwandlung des Dimethylether-Methanol-Wasser-Gemisches in das zunächst wasserhaltige Kohlenwasserstoffgemisch. Vorzugsweise werden dabei Verweilzeiten zwischen 10 Minuten und einer Stunde eingestellt. Beim herkömmlichen MtG-Verfahren ist das nicht möglich, weil die Verweilzeit schon dadurch vorgegeben wird, dass eine bestimmte Oktanzahl erreicht werden soll. Das erfindungsgemäße Verfahren weist diese Einschränkung nicht auf, sondern ermöglicht es vielmehr, die Verweilzeit an die individuellen Gegebenheiten der Rohöl verarbeitenden Raffinerie anzupassen.

So kann bei vergleichsweise kurzen solchen Verweilzeiten, z. B. von zwei bis drei Minuten, nach vollständiger Umsetzung des Dimethylether-Methanol-Gemisches eine Zusammensetzung des Kohlenwasserstoffgemisches von etwa 19 Gew.-% C2-C5 Olefinen, etwa 14 Gew.-% Paraffinen und C6+ Olefinen sowie etwa 9 Gew.-% Aromaten neben Wasser erhalten werden. Bei vergleichsweise langen Verweilzeiten zwischen einer halben Stunde und einer Stunde sinkt der Anteil der C2-C5 Olefine und der jeweilige Anteil der Paraffine und C6+ Olefine sowie der Aromaten steigt an. Der Anteil an Wasser bleibt dabei im Wesentlichen gleich. Bei einer Verweilzeit von etwa einer Stunde kann somit beispielsweise eine Verteilung der Kohlenwasserstoffe von etwa 1 Gew.-% C2-C5 Olefinen, von etwa 17 - 18 Gew.-% Aromaten und von etwa 22 Gew.-% Paraffinen und C6+ Olefinen erhalten werden, wobei der Rest Wasser ist. Mit anderen Worten kann damit nach dem erfindungsgemäßen Verfahren durch die Wahl der Verweilzeit die Fraktion der C2-C5 Olefine entweder als bevorzugte Fraktion oder als Nebenprodukt mit geringem Anteil von ca. 1 Gew.-% eingestellt werden. Entsprechende Verteilungen dazwischen lassen sich ebenso einstellen. Unter dem Begriff "C6+ Olefine" werden hier und im Folgenden insbesondere solche Alkene verstanden, die entweder verzweigt oder unverzweigt sind und 6 oder mehr als 6 Kohlenstoffatome aufweisen, bevorzugt zwischen 6 und 20 Kohlenwasserstoffatome aufweisen, insbesondere zwischen 6 und 15 Kohlenwasserstoffatome aufweisen. Entsprechend sind unter dem Begriff "C2-C5 Olefine" insbesondere Alkene zu verstehen, welche entweder verzweigt oder unverzweigt sind und zwischen 2 und 5 Kohlenstoffatome aufweisen.

Ferner erlaubt es das erfindungsgemäße Verfahren, das durch das Verfahren erhaltene Kohlenwasserstoffgemisch in einem fortgeschrittenen Zwischenschritt der Rohölverarbeitung der Raffinerie zuzuführen. Das Kohlenwasserstoffgemisch muss also nicht zwangsläufig alle Verarbeitungsschritte des zu raffinierenden Rohöls durchlaufen, sondern kann einige dieser Verarbeitungsschritte "überspringen". Auf diese Weise wird die zusätzliche Verarbeitungsbelastung der Rohöl verarbeitenden Raffinerie durch das erfindungsgemäß bereitgestellte Kohlenwasserstoffgemisch noch geringer, als sie ohnehin schon ist.

Ein weiterer Vorteil für die abnehmende Raffinerie ergibt sich daraus, dass im erfindungsgemäßen Verfahren ein vorzugsweise schwefel- und benzolfreies Kohlenwasserstoffgemisch produziert wird, das mit seiner Verarbeitung in der Raffinerie zu einer Senkung des Schwefel- bzw. Benzolgehaltes insbesondere im Benzin beiträgt. Vorzugsweise ist das Kohlenwasserstoffgemisch auch frei von sauerstoffhaltigen Verbindungen.

Erdgas ist ein brennbares Naturgas, das in unterirdischen Lagerstätten vorkommt. Es tritt häufig zusammen mit Erdöl auf, da es auf ähnliche Weise entsteht. Erdgase bestehen hauptsächlich aus Methan, unterscheiden sich aber in ihrer weiteren chemischen Zusammensetzung und je nach Fundstätte erheblich. Häufig enthält Erdgas auch größere Anteile an Ethan (häufig zwischen 1 % und 15 % der molaren Fraktion), Propan (häufig zwischen 1 % und 10 % der molaren Fraktion) und Butan. Weitere Nebenbestandteile sind Schwefelwasserstoff, Stickstoff, Kohlenstoffdioxid und Wasserdampf, daneben Helium und gegebenenfalls elementarer Schwefel und Quecksilber.

Das jeweils erhaltene Rohmethanol wird erfindungsgemäß nachfolgend keiner Destillation unterzogen. Speziell ist bevorzugt, dass das erhaltene Methanol-Wasser-Gemisch (Rohmethanol) ohne zwischengeschaltete Destillation insbesondere direkt im Verfahrensschritt b) in ein Dimethylether-Methanol-WasserGemisch umgewandelt wird, welches anschließend im Verfahrensschritt c) in das wasserhaltige Kohlenwasserstoffgemisch umgewandelt wird.

Dieses Kohlenwasserstoffgemisch hat beispielsweise folgende Zusammensetzung:
- 57 Gew.-% Wasser
- 5 Gew.-% Propan
- 38 Gew.-% Kohlenwasserstoffe, hauptsächlich im Bereich C4 bis C14.

Die Kohlenwasserstoffe bestehen aus Paraffinen, Olefinen und Aromaten.

Erfindungsgemäß umfasst die Umwandlung des Erdgases in das Rohmethanol im obigen Verfahrensschritt a) die folgenden Verfahrensschritte umfasst:
a') Entschwefelung,
b') Sättigung mit Prozesskondensat und Dampf zu einem mit Wasser gesättigten Prozessgas,
c') Umwandeln des Prozessgases in Synthesegas, nämlich ein Gemisch im Wesentlichen bestehend aus Wasserstoff, Kohlendioxid und Kohlenmonoxid,
d') Abkühlen des Synthesegases, vorzugsweise in einem Abhitzesystem, und Komprimieren des Synthesegases mittels eines Kompressors,
e') Gewinnen von Methanol durch katalytische Umwandlung des Synthesegases aus dem Verfahrensschritt d') im Rahmen einer zweistufigen Methanol-Synthese in einer Reaktoranordnung, insbesondere mit einem wassergekühlten Reaktor und einem gasgekühlten Reaktor, und
f') Erhalten des Rohmethanols durch nachfolgende mehrstufige Kondensation des Methanols.

Wie oben bereits angedeutet, weist Erdgas je nach Lagerstelle bzw. Quelle einen Anteil von bis zu 15 % an Stickstoff und einen Anteil an Edelgasen von bis zu 1 % auf. Dieser jeweilige Stickstoffanteil und dieser jeweilige Edelgasanteil wird bei der Umwandlung des Erdgases in das Rohmethanol gemäß dem obigen Verfahrensschritt a), sowie in dem Prozessgas, in dem jeweiligen Synthesegas, in dem weiter unten genannten Spaltgas und in allen weiteren Gasgemischen des Verfahrensschritts a) mitgeführt, ohne dass dies im Weiteren jedesmal ausdrücklich vermerkt wird. Die genannten Zusammensetzungen sind insoweit stets unter Berücksichtigung dieses potentiellen Stickstoffanteils und dieses potentiellen Edelgasanteils zu verstehen. Zusätzlich zu diesem Stickstoffanteil und zu diesem Edelgasanteil also kann das Synthesegas neben Wasserstoff, Kohlendioxid und Kohlenmonoxid noch sehr kleine Mengen z. B. an ungespaltenem Methan aufweisen.

Erfindungsgemäß umfasst das Umwandeln des Prozessgases in Synthesegas im Verfahrensschritt c') das Vorspalten zumindest eines Teiles des Prozessgases in einem Prereformer in ein Spaltgas mit Methan, Wasserstoff, Kohlendioxid und Kohlenmonoxid umfasst. Insbesondere kann dieses Spaltgas ganz oder im Wesentlichen aus Methan, Wasserstoff, Kohlendioxid und Kohlenmonoxid bestehen. Durch eine solche Verwendung eines solchen Prereformers zum Vorspalten kann der Sauerstoffverbrauch bei dem erfindungsgemäßen Verfahren reduziert werden.

Erfindungsgemäß umfasst weiter das Umwandeln des Prozessgases in Synthesegas im Verfahrensschritt c') eine dem Prereformer prozesstechnisch nachgelagerte katalytische Umwandlung eines Verfahrensgasstroms, welcher Verfahrensgasstrom das Spaltgas umfasst, unter erhöhter Temperatur in einem Autothermreformer in das Synthesegas für den Verfahrensschritt d') unter Zusatz von vorgeheiztem Sauerstoff. Unter dem Begriff "Verfahrensgasstrom" ist ein beliebiger Gasstrom im prozesstechnischen Sinne bei dem vorschlagsgemäßen Verfahren zu verstehen, welcher auf dem Prozessgas oder seiner Verarbeitung basiert und welcher bei dem Verfahrensschritt c') vorliegt. Gemäß dieser bevorzugten Ausführung umfasst dieser Verfahrensgasstrom das Spaltgas. Der Verfahrensgasstrom kann auch das Prozessgas umfassen und insbesondere im Wesentlichen aus dem Prozessgas und dem Spaltgas bestehen.

Gemäß einer bevorzugten Ausführungsform wird im Wesentlichen das vollständige Prozessgas aus dem Verfahrensschritt b') dem Prereformer zum Vorspalten zugeführt und das Spaltgas aus dem Prereformer dem Autothermreformer zugeführt. Eine bevorzugte Variante sieht vor, dass das Spaltgas aus dem Prereformer im Wesentlichen vollständig dem Autothermreformer zugeführt wird. Vorzugsweise erfolgt dann die katalytische Umwandlung in dem Autothermreformer bei einem Druck von mindestens 50 bar.

Nach einer weiteren bevorzugten Variante ist vorgesehen, dass das Umwandeln des Prozessgases in Synthesegas im Verfahrensschritt c') eine dem Prereformer prozesstechnisch nachgelagerte Umwandlung des Spaltgases in einem Steamreformer in ein weiteres Synthesegas umfasst. Dieses weitere Synthesegas ist dabei ebenfalls ein Gemisch im Wesentlichen bestehend aus Wasserstoff, Kohlendioxid und Kohlenmonoxid. Das weitere Synthesegas kann aber im Prinzip auch andere Anteile dieser Bestandteile aufweisen als dasjenige Synthesegas, welches in dem Autothermreformer durch Umwandlung entsteht. Vorzugsweise wird das weitere Synthesegas dem Kompressor, insbesondere über das Abhitzesystem, als Synthesegas für den Verfahrensschritt d') zugeführt.

Dieser Steamreformer kann nach einer ersten Variante parallel zu dem Autothermreformer und prozesstechnisch hinter dem Prereformer angeordnet sein. Entsprechend ist es bevorzugt, dass das Spaltgas aus dem Prereformer in einen ersten Spaltgasstrom, welcher dem Autothermreformer zugeführt wird, und einen zweiten Spaltgasstrom, welcher dem Steamreformer zugeführt wird, aufgeteilt wird. Zum Durchführen des folgenden Abkühlens und Komprimierens ist bevorzugt vorgesehen, dass das weitere Synthesegas aus dem Steamreformer und das Synthesegas aus dem Autothermreformer zusammengeführt werden, um das Synthesegas für den Verfahrensschritt d') zu erhalten.

Es ist bevorzugt, dass ein Teilstrom des Prozessgases zur Zuführung zum Prereformer ausgekreist wird, das Spaltgas vom Prereformer vorzugsweise im Wesentlichen vollständig dem Steamreformer zugeführt wird, das weitere Synthesegas aus dem Steamreformer zu dem übrigen Prozessgas zur Vermischung zurückgeführt wird und anschließend dem Autothermreformer zugeführt wird. Vorzugsweise erfolgt die katalytische Umwandlung in dem Autothermreformer bei einem Druck von mindestens 30 bar.

Vorschlagsgemäß wird bei der zweistufigen Methanol-Synthese in dem Verfahrensschritt e') eine erste Teilmenge an Reaktorabgas ausgekreist, die erste Teilmenge an Reaktorabgas im Kreislauf gefahren und dabei auf den Betriebsdruck zur zweistufigen Methanol-Synthese in einem weiteren Kompressor komprimiert. Unter dem Begriff "Reaktorabgas" ist hier und nachfolgend das Gasgemisch gemeint, welches bei der zweistufigen Methanol-Synthese in der Reaktoranordnung anfällt.

Vorschlagsgemäß werden bei der zweistufigen Methanol-Synthese in dem Verfahrensschritt e') eine zweite Teilmenge an Reaktorabgas ausgekreist und einer Pressure-Swing-Anlage (PSA) zugeführt, in welcher aus der zweiten Teilmenge an Reaktorabgas Wasserstoff abgetrennt wird, wobei der abgetrennte Wasserstoff dem Kompressor des Verfahrensschritts d') auf seiner Saugseite zugeführt wird. Eine solche Pressure-Swing-Anlage wird auch als Pressure-Swing-Adsorption-Anlage oder Druckwechsel-Adsorption-Anlage bezeichnet und ist dadurch gekennzeichnet, dass sie ein physikalisches Verfahren zur Trennung von Gasgemischen unter Druck mittels Adsorption bereitstellt.

Vorschlagsgemäß ist weiter vorgesehen, dass ein Synthesegasteilstrom nach dem Komprimieren mittels des Kompressors abgezweigt und ebenfalls der Pressure-Swing-Anlage zugeführt wird. Die Pressure-Swing-Anlage dient somit dazu, die Wasserstoff-Bilanz bei der zweistufigen Methanol-Synthese auszugleichen. Insbesondere ist vorgesehen, dass in der Pressure-Swing-Anlage aus dem Synthesegasteilstrom Wasserstoff abgetrennt wird und dass der abgetrennte Wasserstoff dem Kompressor des Verfahrensschritts d') auf seiner Saugseite zugeführt wird. Vorschlagsgemäß findet die Umwandlung des Rohmethanols in das Dimethylether/Methanol/Wasser-Gemisch des Verfahrensschrittes b) in einem Festbettreaktor statt. Bevorzugt ist auch, dass die Umwandlung des Dimethylether/Methanol/Wasser-Gemisches in das wasserhaltige Kohlenwasserstoffgemisch des Verfahrensschrittes c) in mindestens einem weiteren adiabatisch arbeitenden Reaktor in einem Temperaturbereich von 300 °C bis 450 °C stattfindet. Ebenso ist bevorzugt, dass das im Festbettreaktor anfallende Dimethylether-Methanol-Wasser-Gemisch mit Recyclegas zur Temperatureinstellung versetzt wird, und zwar bevorzugt in den weiteren adiabatisch arbeitenden Reaktoren. Unter dem Begriff "Recyclegas" wird hier und im Folgenden eine rückgeführte Gaskomponente, insbesondere eine im Wesentlichen kohlenwasserstoffhaltige Gaskomponente, aus einer prozesstechnisch nachgeordneten Verarbeitungsstufe, insbesondere aus einer Entgasung gemäß dem Verfahrensschritt d) verstanden. Das zunächst noch wasserhaltige Kohlenwasserstoffgemisch wird nunmehr nach Entgasung und Entwässerung zu einer Rohöl verarbeitenden Raffinerie transportiert, vorzugsweise verpumpt, und zwar insbesondere über eine Rohrleitung, und dort je nach Konfiguration der Raffinerie entweder dem Rohöl oder geeigneten Spaltprodukten zugesetzt.

Hier ist einerseits bevorzugt vorgesehen, dass das gemäß den Verfahrensschritten a) bis d) erhaltene Kohlenwasserstoffgemisch in der Raffinerie prozesstechnisch vor einem Hydrotreater zugesetzt wird. Insbesondere kann es unmittelbar vor einem Hydrotreater der Raffinerie zugesetzt werden. Bei diesem Hydrotreater kann es sich einerseits um einen Hydrotreater handeln, welcher einer "Fluidized Catalytic Cracking" (FCC)-Einheit insbesondere unmittelbar prozesstechnisch vorgelagert ist. Ein solcher, der FCC-Einheit prozesstechnisch vorgelagerter Hydrotreater, kann der Rohöldestillation unmittelbar nachgelagert sein.

Andererseits kann es sich bei dem Hydrotreater um einen Hydrotreater handeln, welcher der FCC-Einheit insbesondere unmittelbar prozesstechnisch nachgelagert ist. Der der FCC-Einheit prozesstechnisch nachgelagerte Hydrotreater ist vorzugsweise einer Benzinabmischung in einem Benzinpool der Raffinerie prozesstechnisch vorgelagert, und zwar vorzugsweise unmittelbar.

Andererseits ist bevorzugt vorgesehen, dass das gemäß den Verfahrensschritten a) bis d) erhaltene Kohlenwasserstoffgemisch in der Raffinerie prozesstechnisch vor einer Rohöldestillation zugesetzt wird. Insbesondere kann es unmittelbar vor einer solchen Rohöldestillation der Raffinerie zugesetzt werden.

Gemäß einem weiteren bevorzugten Ausführungsbeispiel ist vorgesehen, dass das gemäß den Verfahrensschritten a) bis d) erhaltene Kohlenwasserstoffgemisch für das Zuführen zu der Raffinerie verladen wird. Dieses Verladen ist in Abgrenzung zum Verpumpen zu verstehen und kann insbesondere ein Verschiffen sein, bei dem das erhaltene Kohlenwasserstoffgemisch also auf Wasserfahrzeuge, z.

B. Transportschiffe, zum Transport gebracht wird. Alternativ oder zusätzlich kann das erhaltene Kohlenwasserstoffgemisch auch auf Landfahrzeuge, insbesondere Lastkraftwagen, zum Transport verladen werden.

Das vorschlagsgemäße Verfahren zur Herstellung und Nutzung eines Kohlenwasserstoffgemisches für Rohölraffinerien aus Erdgasen kann alternativ auch als Verfahren zur Nutzung von in ein Kohlenwasserstoffgemisch umgewandelten Erdgasen in Rohölraffinerien oder als Verfahren zur Verwertung von Erdgasen durch Umwandlung in ein Kohlenwasserstoffgemisch für Rohölraffinerien beschrieben werden.

Die Erfindung wird nachstehend an zwei Ausführungsbeispielen näher erläutert.

In der Zeichnung zeigt
- Fig. 1: ein Fließschema eines ersten Ausführungsbeispiels des vorschlagsgemäßen Verfahrens,
- Fig. 2: ein Fließschema eines zweiten Ausführungsbeispiels des vorschlagsgemäßen Verfahrens und
- Fig. 3: eine Darstellung gemessener Bestandteile des wasserhaltigen Kohlenwasserstoffgemisches aus der Umwandlung des Dimethylether-Methanol-Wasser-Gemisches in Abhängigkeit von der Verweilzeit.

Ein Erdgas (350.000 Nm³/h) mit nachfolgender Zusammensetzung

| | | |
|---|---|---|
| - | Stickstoff | 1,5 % des Volumens |
| - | Methan | 92 % des Volumens |
| - | Ethan | 3,5 % des Volumens |
| - | Propan | 1,5 % des Volumens |
| - | Höhere Kohlenwasserstoffe | 1,0 % des Volumens |
| - | Schwefel | 50 ppm |

wird in einer in der Nähe einer Rohöl verarbeitenden Raffinerie 25 errichteten Chemieanlage, welche in zwei Ausführungsbeispielen nachfolgend näher beschrieben wird, wie folgt in ein Kohlenwasserstoffgemisch umgewandelt:

### Ausführungsbeispiel 1:

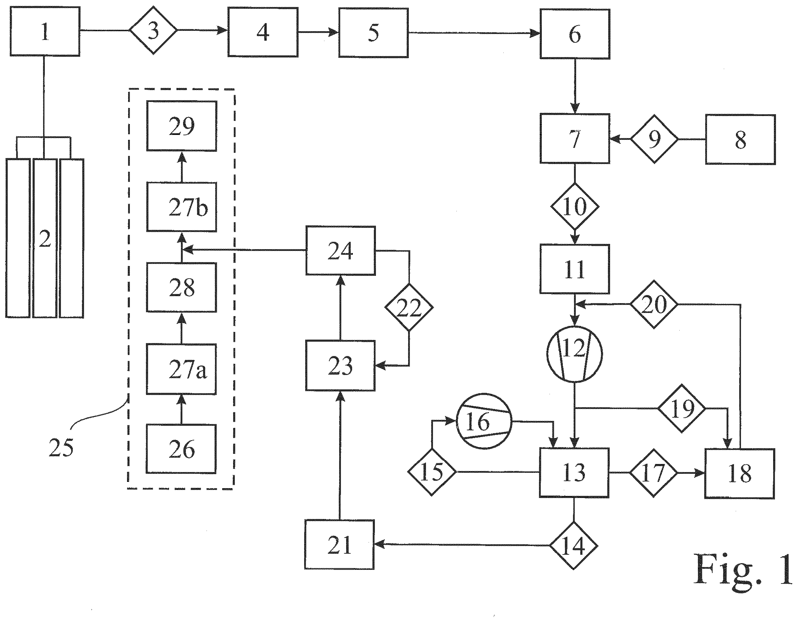

Die Fig. 1 zeigt für das erste Ausführungsbeispiel eine zentrale Ölaufarbeitung 1 für aus unterschiedlichen Bohrlöchern 2 stammendes Rohöl. Das obige Erdgas 3 fällt dabei als Erdölbegleitgas an. Dieses Erdgas 3 wird bei einem Druck von 70 bar zunächst bei einer Temperatur von 375 °C über einem Zinkoxidbett entschwefelt (Entschwefelungseinheit 4), danach mit Prozesskondensat und Dampf zu einem mit Wasser gesättigten Prozessgas gesättigt (Sättiger 5) und nach Einstellung eines Dampf/Kohlenstoffverhältnisses von 1,0 im Prereformer 6, einem adiabatisch arbeitenden Katalysereaktor, bei 480 °C in ein Spaltgas aus Methan, Wasserstoff, Kohlendioxid und Kohlenmonoxid vorgespalten.

Nach weiterer Aufheizung auf 630 bis 650 °C wird das Spaltgas einem Autothermreformer 7 zugeführt. Bei dem Autothermreformer 7 handelt es sich ebenfalls um einen adiabatisch arbeitenden Katalysereaktor, in welchem durch Zusatz von auf 230°C vorgeheiztem Sauerstoff 9, der in einer Luftzerlegungsanlage 8 gewonnen wird, bei 1030 °C ein Synthesegas 10 erzeugt wird, das aus Wasserstoff, Kohlenmonoxid und Kohlendioxid besteht und nur noch eine sehr kleine Menge ungespaltenes Methan enthält. Dieses Synthesegas 10 wird in einem Abhitzesystem 11 gekühlt.

Über verschiedene Stufen, die zur Dampferzeugung bzw. Erwärmung verschiedener Gas/Produktströme genutzt werden, wird das nunmehr bei 55 bar anliegende und gekühlte Synthesegas mit einem Kompressor 12 auf 75 bar komprimiert. Nachfolgend wird in einem dualen System, bestehend aus einer Reaktoranordnung 13 mit einem wassergekühlten und einem gasgekühlten Reaktor, Synthesegas katalytisch im Temperaturbereich von 220 bis 260 °C in Methanol umgewandelt und durch Kondensation ein Rohmethanol 14 mit folgender Zusammensetzung erhalten:

| | | |
|---|---|---|
| - | Methanol | 83 Gew.-% |
| - | Kohlendioxid | 3,6 Gew.-% |
| - | Wasser | 11,7 Gew.-% |
| - | Methan | 1,5 Gew.-% |
| - | Höhere Kohlenwasserstoffe | 0,1 Gew.-% |
| - | Höhere Alkohole | 0,1 Gew.-% |

Während der Methanolsynthese wird eine Teilmenge an Reaktorabgasgas 15 über eine Kreislaufleitung im Kreislauf gefahren und dabei mittels eines weiteren Kompressors 16 auf den erforderlichen Druck gebracht.

Aufgrund der im Synthesegas 10 enthaltenen Verunreinigungen wird eine zweite Teilmenge an Reaktorabgas 17 als Purgegas ausgekreist und über eine Pressure-Swing-Anlage (PSA) 18 gefahren. Dieser PSA 18 wird auch unter hohem Druck ein Synthesegasteilstrom 19 zugeführt, der nach der Druckerhöhung mittels des Kompressors 12 abgezweigt wird. Der in der PSA 18 erzeugte Wasserstoff 20 wird auf der Saugseite des Kompressors 12 in den Synthesegasstrom zurückgeführt.

Das Rohmethanol 14 (503 t/h) wird nachfolgend in einem Festbettreaktor 21 (DME-Reaktor) katalytisch in ein DME (Dimethylether/Methanol/Wasser)-Gemisch umgewandelt. Das Reaktionsprodukt aus dem DME-Reaktor wird mit Recyclegas 22 zur Temperatureinstellung versetzt und nachfolgend in weiteren adiabatisch arbeitenden Reaktoren 23 im Temperaturbereich von 320 bis 420 °C und bei einer Verweilzeit von 60 Minuten in ein wasserhaltiges Kohlenwasserstoff-Gemisch umgewandelt. Aus den eingesetzten 503 t/h Methanol entstehen dabei 191 t Kohlenwasserstoffe und 312 t Wasser. Dieses wässrige Kohlenwasserstoffgemisch wird abschließend in einer Nachbereitungseinheit 24 entgast und entwässert.

Das entwässerte und entgaste Kohlenwasserstoffgemisch (191 t/h) wird in eine naheliegende und Rohöl verarbeitende Raffinerie 25 verpumpt. Die hier prozesstechnisch relevanten Verarbeitungsstufen dieser Raffinerie 25 umfassen, in dieser Reihenfolge, eine Rohöldestillation 26, einen ersten Hydrotreater 27a, eine FCC-Einheit 28, einen zweiten Hydrotreater 27b und einen Benzinpool 29 für die Benzinabmischung. In diesem Ausführungsbeispiel wird das Kohlenwasserstoffgemisch vor dem zweiten Hydrotreater 27b zugesetzt, welcher der "Fluidized Catalytic Cracking" (FCC)-Einheit 28 prozesstechnisch nachgelagert ist. Der Vorteil dieser Einspeisung besteht darin, dass die eingespeisten Kohlenwasserstoffe als vorzugsweise schwefel- und benzolfreie Komponente die Qualität des produzierten Benzins verbessern. Praktisch 100 % des Kohlenwasserstoffgemisches werden letztlich in Benzin umgewandelt. Damit werden die im Kohlenwasserstoffgemisch enthaltenen Kohlenwasserstoffe dem Benzinpool 29 zugesetzt. Die gewählte Verweilzeit in den adiabatisch arbeitenden Reaktoren 23 führt dabei zu einem Gemisch, das etwa 65 % Paraffine und C6+ Olefine sowie 35 % Aromaten (vorwiegend Toluol und Xylol) enthält.

### Ausführungsbeispiel 2:

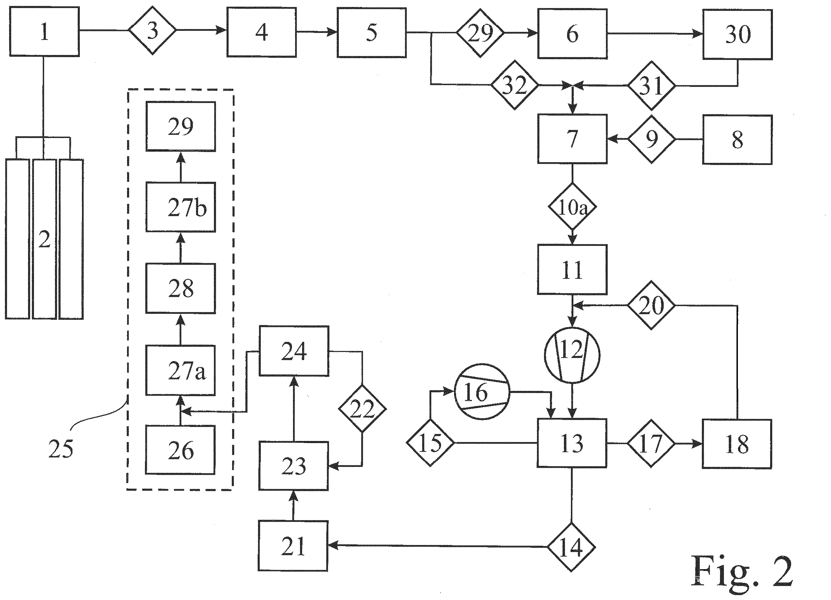

Das in Fig. 2 gezeigte zweite Ausführungsbeispiel entspricht grundsätzlich dem obigen, mit Bezug auf die Fig. 1 beschriebenen ersten Ausführungsbeispiel, unterscheidet sich jedoch in den folgenden Merkmalen:
Nach dem Sättiger 5 wird über eine erste Leitung 29 ein erster Teilstrom von etwa 40 % des mit Wasser gesättigten, entschwefelten und mit Dampf gemischten Prozessgases mit einer Temperatur von etwa 480 °C dem Prereformer 6 zugeführt. In diesem wird das Prozessgas in ein Spaltgas aus Methan, Wasserstoff, Kohlendioxid und Kohlenmonoxid vorgespalten.

Nach weiterer Aufheizung auf 520 °C gelangt dieses Spaltgas in einen Steamreformer 30, speziell einem außenbeheizten Röhrenreaktor mit Nickel-Katalysator, und wird in diesem in ein weiteres Synthesegas 31, und zwar ein Gemisch aus Wasserstoff, Kohlenmonoxid und Kohlendioxid, umgewandelt.

Dieses weitere Synthesegas 31 wird zu dem in der anderen, zweiten Leitung 32 geführten Teilstrom (ca. 60%) des mit Wasser gesättigten, entschwefelten und mit Dampf gemischten Prozessgases, das nach dem Sättiger 5 anfällt, zurückgeführt, mit diesem vermischt und mit einer Mischtemperatur von 670°C dem Autothermreformer 7 zugeführt.

Im Autothermreformer 7, einem adiabatisch arbeitenden Katalysereaktor, wird das Mischgas durch Zusatz von auf 240 °C erwärmtem Sauerstoff 9, der in einer Luftzerlegungsanlage 8 gewonnen wird, bei 980 °C vollständig in ein zweites Synthesegas 10a umgewandelt, das nur noch eine sehr kleine Menge ungespaltenes Methan enthält. Dieses zweite Synthesegas 10a wird in dem Abhitzesystem 11 gekühlt.

Das mit einem Druck von 32 bar anliegende zweite Synthesegas 10a wird nachfolgend in zu dem Synthesegas 10 des ersten Ausführungsbeispiels analoger Weise, wie in Beispiel 1 angegeben, weiterbehandelt, um ein wasserfreies Kohlenwasserstoffgemisch mit der oben beschriebenen Zusammensetzung herzustellen, mit dem Unterschied, dass nach dem Kompressor 12 kein Synthesegasteilstrom 19 abgezweigt und der Pressure-Swing-Anlage (PSA) 18 zugeführt wird. Mit dieser Verfahrensweise ist es möglich, im Vergleich zu der Verfahrensweise gemäß Beispiel 1 den Gasverbrauch, insbesondere den Sauerstoffverbrauch, für die Herstellung des Kohlenwasserstoffgemisches um 10 % zu reduzieren.

Ein weiterer Unterschied des zweiten Ausführungsbeispiels besteht darin, dass das entwässerte und entgaste Kohlenwasserstoffgemisch nach dem Verpumpen zur naheliegenden Raffinerie 25 vor dem ersten Hydrotreater 27a und damit unmittelbar nach der Rohöldestillation 28 zugesetzt wird. Dabei kann der prozesstechnische Punkt der Zusetzung des Kohlenwasserstoffgemisches zwischen dem ersten und dem zweiten Ausführungsbeispiel auch getauscht werden.

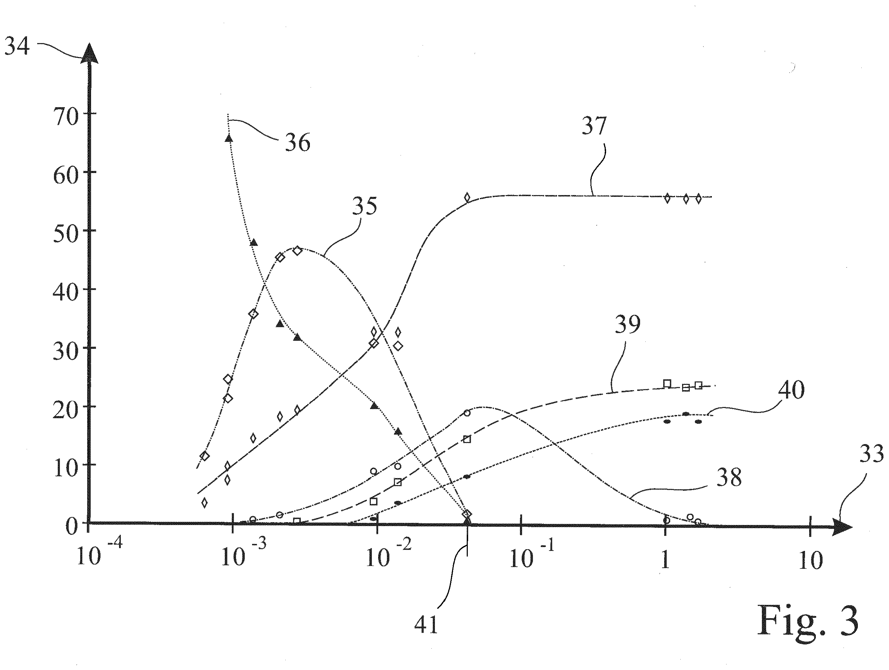

Die Figur 3 zeigt eine Darstellung gemessener Bestandteile des wasserhaltigen Kohlenwasserstoffgemisches aus der Umwandlung des Dimethylether-Methanol-Wasser-Gemisches in Abhängigkeit von der Verweilzeit. Diese Darstellung basiert auf der Interpolation einzelner Messpunkte. Speziell ist auf der Abszisse 33 die Verweilzeit in Stunden logarithmisch aufgetragen, während auf der Ordinate 34 die Produktselektivität in Gew.-% angezeigt wird. Zu Beginn des Prozesses liegen ausschließlich Dimethylether 35 und Methanol 36 sowie ein geringer Anteil Wasser 37 vor, wobei bei fortschreitender Verweilzeit und damit fortschreitender Umwandlung der Anteil an Wasser 37 stark steigt und die jeweiligen Anteile der dann zunehmend gebildeten C2-C5 Olefine 38, Paraffine und C6+ Olefine (hier gemeinsam dargestellt) 39, sowie der Aromaten 40 zunächst langsam anwachsen. Ebenso verringert sich der Anteil der Reaktanten Dimethylether 35 und Methanol 36.

Ungefähr nach der Umwandlung des gesamten Dimethylethers 35 und des Methanols 36 erreicht der Anteil des Wassers 37 einen konstanten Wert und der Anteil der C2-C5 Olefine 38 erreicht sein Maximum. Bei längerer Verweilzeit über diesen Punkt der vollständigen Umwandlung 41 der Reaktanten hinaus nimmt der Anteil der C2-C5 Olefine 38 wieder ab zugunsten der Aromaten 40 sowie der Paraffine und C6+ Olefine 39. Dies setzt sich fort bis zu einer nahezu vollständigen Umwandlung der C2-C5 Olefine 38 - also der kurzkettigen Olefine - in Aromaten 40 einerseits sowie in Paraffine und C6+ Olefine 39 andererseits.

## Patentansprüche

1. Verfahren zur Herstellung und Nutzung eines Kohlenwasserstoffgemisches für Rohölraffinerien aus Erdgasen (3), wobei das Kohlenwasserstoffgemisch wasserfrei ist und aus Erdgas mit folgenden Verfahrensschritten hergestellt wird:
a) Umwandlung des Erdgases in ein Methanol/Wasser-Gemisch (Rohmethanol) (14) ohne nachfolgende Destillation,
b) katalytische Umwandlung des Rohmethanols (14) in ein Dimethylether/Methanol/Wasser-Gemisch,
c) Umwandlung des Dimethylether/Methanol/Wasser-Gemisches in ein wasserhaltiges Kohlenwasserstoffgemisch, vorwiegend bestehend aus Paraffinen, C6+ Olefinen und Aromaten, insbesondere methylierten Benzolen,
d) Entgasung und Entwässerung des Kohlenwasserstoffgemisches,
wobei die Umwandlung des Erdgases (3) in das Rohmethanol (14) im Verfahrensschritt a) die folgenden Verfahrensschritte umfasst:
a') Entschwefelung,
b') Sättigung mit Prozesskondensat und Dampf zu einem mit Wasser gesättigten Prozessgas,
c') Umwandeln des Prozessgases in Synthesegas (10), nämlich ein Gemisch im Wesentlichen bestehend aus Wasserstoff, Kohlendioxid und Kohlenmonoxid, umfassend das Vorspalten zumindest eines Teils des Prozessgases in einem Prereformer (6) in ein Spaltgas mit Methan, Wasserstoff, Kohlendioxid und Kohlenmonoxid und umfassend eine dem Prereformer (6) prozesstechnisch nachgelagerte katalytische Umwandlung eines Verfahrensgasstroms, welcher Verfahrensgasstrom das Spaltgas umfasst, unter erhöhter Temperatur in einem Autothermreformer (7), in das Synthesegas (10) für den Verfahrensschritt d') unter Zusatz von vorgeheiztem Sauerstoff (9),
d') Abkühlen des Synthesegases (10) und Komprimieren des Synthesegases (10) mittels eines Kompressors (12),
e') Gewinnen von Methanol durch katalytische Umwandlung des Synthesegases (10) aus dem Verfahrensschritt d') im Rahmen einer zweistufigen Methanol-Synthese in einer Reaktoranordnung (13) und
f') Erhalten des Rohmethanols (14) durch nachfolgende mehrstufige Kondensation des Methanols,
wobei bei der zweistufigen Methanol-Synthese in dem Verfahrensschritt e') eine erste Teilmenge an Reaktorabgas (15) ausgekreist, im Kreislauf gefahren und dabei auf den Betriebsdruck zur zweistufigen Methanol-Synthese in einem weiteren Kompressor (16) komprimiert wird,
wobei bei der zweistufigen Methanol-Synthese in dem Verfahrensschritt e') eine zweite Teilmenge an Reaktorabgas (17) ausgekreist und einer Pressure-Swing-Anlage (18) zugeführt wird, in welcher aus der zweiten Teilmenge an Reaktorabgas (17) Wasserstoff abgetrennt wird, wobei der abgetrennte Wasserstoff (20) dem Kompressor (12) des Verfahrensschritts d') auf seiner Saugseite zugeführt wird,
wobei ein Synthesegasteilstrom (19) nach dem Komprimieren mittels des Kompressors (12) abgezweigt und ebenfalls der Pressure-Swing-Anlage (18) zugeführt wird,
wobei die Umwandlung des Rohmethanols (14) in das Dimethylether/Methanol/Wasser-Gemisch des Verfahrensschrittes b) in einem Festbettreaktor (21) stattfindet, wobei das im Festbettreaktor (21) anfallende Dimethylether/Methanol/Wasser-Gemisch mit Recyclegas (22) zur Temperatureinstellung versetzt wird,
und wobei das gemäß den Verfahrensschritten a) bis d) erhaltene Kohlenwasserstoffgemisch einer, insbesondere naheliegenden, Rohöl verarbeitenden Raffinerie (25) zugeführt wird, vorzugsweise verpumpt wird, und dort in insbesondere spezifikationsgerechte Produkte, vorzugsweise in Benzin und/oder Aromaten, umgewandelt wird.

2. Verfahren nach Anspruch 1, **dadurch gekennzeichnet, dass** im Wesentlichen das vollständige Prozessgas aus dem Verfahrensschritt b') dem Prereformer (6) zum Vorspalten zugeführt wird und das Spaltgas aus dem Prereformer (6), vorzugsweise im Wesentlichen vollständig, dem Autothermreformer (7) zugeführt wird, insbesondere, wobei die katalytische Umwandlung in dem Autothermreformer (7) bei einem Druck von mindestens 50 bar erfolgt.

3. Verfahren nach Anspruch 1 oder 2, **dadurch gekennzeichnet, dass** das Umwandeln des Prozessgases in Synthesegas (10) im Verfahrensschritt c') eine dem Prereformer (6) prozesstechnisch nachgelagerte Umwandlung des Spaltgases in einem Steamreformer (30) in ein weiteres Synthesegas (31) umfasst, vorzugsweise, dass das weitere Synthesegas (31) dem Kompressor (12), vorzugsweise über das Abhitzesystem (11), als Synthesegas für den Verfahrensschritt d') zugeführt wird.

4. Verfahren nach Anspruch 3, **dadurch gekennzeichnet, dass** das Spaltgas aus dem Prereformer (6) in einen ersten Spaltgasstrom, welcher dem Autothermreformer (7) zugeführt wird, und einen zweiten Spaltgasstrom, welcher dem Steamreformer (30) zugeführt wird, aufgeteilt wird, vorzugsweise, wobei das weitere Synthesegas (31) aus dem Steamreformer (30) und das Synthesegas (10) aus dem Autothermreformer (7) zusammengeführt werden, um das Synthesegas für den Verfahrensschritt d') zu erhalten.

5. Verfahren nach Anspruch 3, **dadurch gekennzeichnet, dass** ein Teilstrom des Prozessgases zur Zuführung zum Prereformer (6) ausgekreist wird, das Spaltgas vom Prereformer (6) vorzugsweise im Wesentlichen vollständig dem Steamreformer (30) zugeführt wird, das weitere Synthesegas (31) aus dem Steamreformer (30) zu dem übrigen Prozessgas zur Vermischung zurückgeführt wird und anschließend dem Autothermreformer (7) zugeführt wird, vorzugsweise, wobei die katalytische Umwandlung in dem Autothermreformer (7) bei einem Druck von mindestens 30 bar erfolgt.

6. Verfahren nach einem der Ansprüche 1 bis 5, **dadurch gekennzeichnet, dass** in der Pressure-Swing-Anlage (18) aus dem Synthesegasteilstrom (19) Wasserstoff abgetrennt wird und dass der abgetrennte Wasserstoff (20) dem Kompressor (12) des Verfahrensschritts d') auf seiner Saugseite zugeführt wird.

7. Verfahren nach einem der Ansprüche 1 bis 6, **dadurch gekennzeichnet, dass** die Umwandlung des Dimethylether/Methanol/Wasser-Gemisches in das wasserhaltige Kohlenwasserstoffgemisch des Verfahrensschrittes c) in mindestens einem weiteren adiabatisch arbeitenden Reaktor (23) in einem Temperaturbereich von 300 °C bis 450 °C stattfindet.

8. Verfahren nach einem der Ansprüche 1 bis 7, **dadurch gekennzeichnet, dass** das gemäß den Verfahrensschritten a) bis d) erhaltene Kohlenwasserstoffgemisch in der Raffinerie (25) prozesstechnisch insbesondere unmittelbar vor einem Hydrotreater (26) zugesetzt wird.

9. Verfahren nach einem der Ansprüche 1 bis 8, **dadurch gekennzeichnet, dass** das gemäß den Verfahrensschritten a) bis d) erhaltene Kohlenwasserstoffgemisch in der Raffinerie (25) prozesstechnisch insbesondere unmittelbar vor einer Rohöldestillation (28) zugesetzt wird.

10. Verfahren nach einem der Ansprüche 1 bis 9, **dadurch gekennzeichnet, dass** das gemäß den Verfahrensschritten a) bis d) erhaltene Kohlenwasserstoffgemisch für das Zuführen zu der Raffinerie (25) verladen, vorzugsweise verschifft, wird.

## Claims

1. Method for the production and use of a hydrocarbon mixture from natural gases (3) for crude oil refineries, wherein the hydrocarbon mixture is anhydrous and is produced from natural gas using the following method steps:
a) converting the natural gas into a methanol/water mixture (crude methanol) (14) without subsequent distillation,
b) catalytic conversion of the crude methanol (14) to a dimethyl ether/methanol/water mixture,
c) conversion of the dimethyl ether/methanol/water mixture to a hydrous hydrocarbon mixture predominantly consisting of paraffins, C6+ olefins and aromatics, in particular methylated benzenes,
d) degassing and dehydration of the hydrocarbon mixture,
wherein the conversion of the natural gas (3) into the crude methanol (14) in method step (a) comprises the following method steps:
a') desulfurizing,
b') saturating with process condensate and steam to form a process gas saturated with water,
c') converting the process gas into synthesis gas (10), namely a mixture consisting substantially of hydrogen, carbon dioxide and carbon monoxide, comprising the prior cracking of at least a portion of the process gas in a pre-reformer (6) into a cracked gas with methane, hydrogen, carbon dioxide and carbon monoxide, and comprising a catalytic conversion of a process gas flow, performed downstream from the pre-reformer (6) in the process, wherein said process gas flow comprises the cracked gas, into the synthesis gas (10) under elevated temperature in an autothermal reformer (7) for method step d'), with the addition of preheated oxygen (9),
d') cooling the synthesis gas (10) and compressing the synthesis gas (10) via a compressor (12),
e') yielding methanol by catalytic conversion of the synthesis gas (10) from the method step d') within the scope of a two-stage methanol synthesis in a reactor arrangement (13), and
f') obtaining the crude methanol (14) by the subsequent multi-stage condensation of the methanol,
wherein in the two-stage methanol synthesis, in the method step e') a first portion of reactor effluent gas (15) is branched out, circulated, and in a second compressor (16) compressed in the process to an operating pressure for the two-stage methanol synthesis,
wherein in the two-stage methanol synthesis, in the method step e') a second portion of reactor effluent gas (17) is branched out and fed to a pressure-swing arrangement (18), in which hydrogen is separated from the second portion of reactor effluent gas (17), wherein the separated hydrogen (20) is fed to the compressor (12) of method step d') at its suction side,
wherein a partial flow of synthesis gas (19) is branched off after compression via the compressor (12) and also fed to the pressure swing arrangement (18),
wherein the conversion of the crude methanol (14) into the dimethyl ether/methanol/water mixture of the method step b) occurs in a fixed bed reactor (21), wherein the dimethyl ether/methanol/water mixture developing in the fixed bed reactor (21) is mixed with recycle gas (22) for temperature adjustment,
and wherein the hydrocarbon mixture yielded according to the method steps a) to d) is fed, preferably pumped, to a refinery (25), particularly a nearby refinery, for processing crude oil, where it is converted particularly into specification-conforming products, preferably gasoline and/or aromatics.

2. Method according to Claim 1, **characterized in that** essentially the entire process gas from the method step b') is fed to the pre-reformer (6) for pre-cracking, and preferably essentially all of the cracked gas is fed, from the pre-reformer (6) to the auto-thermal reformer (7), wherein in particular the catalytic conversion occurs in the auto-thermal reformer (7) at a pressure of at least 50 bar.

3. Method according to Claim 1 or 2, **characterized in that** the conversion of the process gas into synthesis gas (10) in the method step c') comprises a conversion of the cracked gas in a steam reformer (30) into a further synthesis gas (31) procedurally downstream the pre-reformer (6), preferably that the further synthesis gas (31) is fed to the compressor (12) particularly via the waste heat boiler system (11), as the synthesis gas for the method step d').

4. Method according to Claim 3, **characterized in that** the cracked gas from the pre-reformer (6) is divided into a first cracked gas flow, which is fed to the auto-thermal reformer (7), and a second cracked gas flow, which is fed to the steam reformer (30), wherein the further synthesis gas (31) from the steam reformer (30) and the synthesis gas (10) of the auto-thermal reformer (7) are preferably combined in order to yield the synthesis gas for the method step d').

5. Method according to Claim 3, **characterized in that** a partial flow of the process gas is branched out for feeding the pre-reformer (6), the cracked gas from the pre-reformer (6) is preferably fed substantially in its entirety to the steam reformer (30), the further synthesis gas (31) is returned from the steam reformer (30) to the remaining process gas for mixing, and it is then fed to the auto-thermal reformer (7), preferably, wherein the catalytic conversion occurs in the auto-thermal reformer (7) at a pressure of at least 30 bar.

6. Method according to any one of Claims 1 to 5, **characterized in that** hydrogen is branched out of the partial flow of the synthesis gas (19) in the pressure swing plant (18), and that the separated hydrogen (20) is fed to the compressor (12) of method step d') at its suction side.

7. Method according to any one of Claims 1 to 6, **characterized in that** the conversion of the dimethyl ether/methanol/water mixture into the aqueous hydrocarbon mixture of the method step c) occurs in at least one further adiabatically operating reactor (23) at a temperature range from 300 °C to 450 °C.

8. Method according to any one of Claims 1 to 7, **characterized in that** the hydrocarbon mixture yielded according to the processing steps a) to d) is added in the refinery (25) particularly immediately upstream a hydro-treater (26).

9. Method according to any one of Claims 1 to 8, **characterized in that** the hydrocarbon mixture yielded according to method steps a) to d) is added in the refinery (25) particularly immediately procedurally upstream a crude oil distillation (28).

10. Method according to any one of Claims 1 to 9, **characterized in that** the hydrocarbon mixture yielded according to method steps a) to d) is loaded, preferably to water craft, for delivery to the refinery (25).

## Revendications

1. Procédé, destiné à produire et à utiliser un mélange d'hydrocarbures pour des raffineries de pétrole brut à partir de gaz naturels (3), le mélange d'hydrocarbures étant exempt d'eau et étant produit à partir de gaz naturel, avec les étapes de procédé suivantes, consistant dans :
a) la transformation du gaz naturel en un mélange eau/méthanol (méthanol brut) (14) sans distillation consécutive,
b) la transformation catalytique du méthanol brut (14) en un mélange eau/méthanol/éther diméthylique,
c) la transformation du mélange eau/méthanol/éther diméthylique en un mélange d'hydrocarbures contenant de l'eau, constitués majoritairement de paraffines, d'oléfines C6+ et d'aromates, notamment de benzènes méthylés,
d) le dégazage et la déshydratation du mélange d'hydrocarbures,
la transformation du gaz naturel (3) en le méthanol brut (14) à l'étape de procédé a) comprenant les étapes suivantes consistant dans :
a') la désulfurisation,
b') la saturation avec du condensat de procédé et de la vapeur en un gaz de processus saturé à l'eau,
c') la transformation du gaz de processus en un gaz de synthèse (10), à savoir un mélange consistant sensiblement dans de l'hydrogène, du dioxyde de carbone et du monoxyde de carbone, comprenant la dissociation préalable d'au moins une partie du gaz de processus dans un préreformeur (6) en un gaz de craquage avec du méthane, de l'hydrogène, du dioxyde de carbone et du monoxyde de carbone et comprenant une transformation catalytique en aval du préreformeur (6) dans la technique du processus d'un flux de gaz de procédé, lequel flux de gaz de procédé comprend le gaz de craquage à température élevée dans un reformeur autotherme (7), dans le gaz de synthèse (10) pour l'étape de procédé d') en ajoutant de l'oxygène (9) préchauffé,
d') le refroidissement du gaz de synthèse (10) et la compression du gaz de synthèse (10) au moyen d'un compresseur (12),
e') la récupération de méthanol par transformation catalytique du gaz de synthèse (10) provenant de l'étape de procédé d') dans le cadre d'une synthèse du méthanol en deux étapes, dans un ensemble de réacteurs (13) et
f') l'obtention du méthanol brut (14) par condensation successive en plusieurs étapes du méthanol,
lors de la synthèse du méthanol en deux étapes dans l'étape de procédé e'), une première quantité partielle du gaz de sortie (15) du réacteur étant sortie du circuit, acheminée dans le circuit et comprimée à cet effet à la pression de service dans un autre compresseur (16), pour la synthèse du méthanol en deux étapes,
lors de la synthèse du méthanol en deux étapes, à l'étape de procédé e'), une deuxième quantité partielle de gaz de sortie (17) du réacteur étant sortie du circuit et alimentée vers une installation de modulation en pression (18), dans laquelle, à partir de la deuxième quantité partielle de gaz de sortie (17) du réacteur est séparé l'hydrogène, l'hydrogène (20) séparé étant alimenté vers le compresseur (12) de l'étape de procédé d') sur le côté aspiration de celui-ci,
après la compression au moyen du compresseur (12), un flux partiel (19) de gaz de synthèse étant dévié et également alimenté vers l'installation de modulation en pression (18),
la transformation du méthanol brut (14) dans le mélange eau/méthanol/éther diméthylique de l'étape de procédé b) ayant lieu dans un réacteur à lit fixe (21), le mélange eau/méthanol/éther diméthylique produit dans le réacteur à lit fixe (21) étant mélangé avec du gaz recyclé (22) pour le réglage de la température,
et le mélange d'hydrocarbures obtenu selon les étapes de procédé a) à d) étant alimenté, de préférence pompé vers une raffinerie (25) notamment proche, traitant du pétrole brut, et y étant transformé en produits notamment conformes aux spécifications, de préférence en essence et / ou en aromates.

2. Procédé selon la revendication 1, **caractérisé en ce que** sensiblement la totalité du gaz de processus provenant de l'étape de procédé b') est alimentée vers le préreformeur (6) pour la dissociation préalable et le gaz de craquage provenant du préreformeur (6) est alimenté, de préférence sensiblement en totalité vers le reformeur autotherme (7), notamment la transformation catalytique dans le reformeur autotherme (7) s'effectuant à une pression d'au moins 50 bar.

3. Procédé selon la revendication 1 ou 2, **caractérisé en ce que** la transformation du gaz de processus en gaz de synthèse (10) à l'étape de procédé c') comprend une transformation placé en aval du préreformeur (6) selon la technique du processus du gaz de craquage dans un reformeur à vapeur (30) en un gaz de synthèse (31) supplémentaire, de préférence, **en ce que** le gaz de synthèse (31) supplémentaire est alimenté vers le compresseur (12), de préférence via le système de chaleur perdue (11), en tant que gaz de synthèse pour l'étape de procédé d').

4. Procédé selon la revendication 3, **caractérisé en ce que** le gaz de craquage issu du préreformeur (6) est divisé en un premier flux de gaz de craquage, lequel est alimenté vers le reformeur autotherme (7) et en un deuxième flux de gaz de craquage, lequel est alimenté vers le reformeur à vapeur (30), de préférence, le gaz de synthèse (31) supplémentaire issu du reformeur à vapeur (30) et le gaz de synthèse (10) issu du reformeur autotherme (7) étant réunis, pour obtenir le gaz de synthèse pour l'étape de procédé d').

5. Procédé selon la revendication 3, **caractérisé en ce qu'**un flux partiel du gaz de processus est sorti du circuit pour être alimenté vers le préreformeur (6), le gaz de craquage issu du préreformeur (6) est alimenté de manière sensiblement totale vers le reformeur à vapeur (30), le gaz de synthèse (31) supplémentaire issu du reformeur à vapeur (30) est alimenté vers le gaz de processus restant, pour être mélangé et par la suite, est alimenté vers le reformeur autotherme (7), de préférence, la transformation catalytique dans le reformeur autotherme (7) s'effectuant à une pression d'au moins 30 bar.

6. Procédé selon l'une quelconque des revendications 1 à 5, **caractérisé en ce que** dans l'installation de modulation en pression (18), de l'hydrogène est séparé du flux partiel (19) de gaz de synthèse et **en ce que** l'hydrogène (20) séparé est alimenté vers le compresseur (12) de l'étape de procédé d') sur le côté aspiration de celui-ci.

7. Procédé selon l'une quelconque des revendications 1 à 6, **caractérisé en ce que** la transformation du mélange eau/méthanol/éther diméthylique en le mélange d'hydrocarbures contenant de l'eau de l'étape de procédé c) a lieu dans au moins un réacteur (23) supplémentaire à fonctionnement adiabatique, dans une plage de température de 300 °C à 450 °C.

8. Procédé selon l'une quelconque des revendications 1 à 7, **caractérisé en ce que** dans la raffinerie (25), le mélange d'hydrocarbures obtenu selon les étapes de procédé a) à d) est ajouté selon la technique du processus notamment directement à l'avant d'un hydrotraiteur (26).

9. Procédé selon l'une quelconque des revendications 1 à 8, **caractérisé en ce que** dans la raffinerie (25), le mélange d'hydrocarbures obtenu selon les étapes de procédé a) à d) est ajouté selon la technique du processus notamment directement avant une distillation (28) de pétrole brut.

10. Procédé selon l'une quelconque des revendications 1 à 9, **caractérisé en ce que** le mélange d'hydrocarbures obtenu selon les étapes de procédé a) à d) est chargé, notamment transbordé pour l'alimentation vers la raffinerie (25).
